Europäisches Patentamt

European Patent Office

Office européen des brevets

(11) Veröffentlichungsnummer: **0 362 494**

**A1**

(12) **EUROPÄISCHE PATENTANMELDUNG**

(21) Anmeldenummer: **89112699.7**

(22) Anmeldetag: **12.07.89**

(51) Int. Cl.5: **A61M 19/00 , A61M 25/01 , A61M 5/14**

(30) Priorität: **09.09.88 DE 8811408 U**

(43) Veröffentlichungstag der Anmeldung:
**11.04.90 Patentblatt 90/15**

(84) Benannte Vertragsstaaten:
**AT BE CH DE ES FR GB IT LI LU NL SE**

(71) Anmelder: **B. Braun Melsungen AG**
**Carl-Braun Strasse**
**D-3508 Melsungen(DE)**

(72) Erfinder: **Biscoping, Jürgen, Prof. Dr.**
**Abt. f. Anästhesiologie Klinikstrasse 29**
**D-6300 Giessen(DE)**
Erfinder: **Summerer, Marie-Louise**
**Buchenhain 15**
**D-3501 Körle(DE)**
Erfinder: **Wiegel, Heinz, Dipl.-Ing.**
**Eisfeldstrasse 10**
**D-6445 Alheim(DE)**
Erfinder: **Witt, Hans-Hinrich, Dr.**
**Buchenhain 15**
**D-3501 Körle(DE)**

(74) Vertreter: **Selting, Günther et al**
**Patentanwälte Von**
**Kreisler-Schönwald-Selting-Fues-Dallmeyer--**
**Werner-Dalmeyer Deichmannhaus**
**D-5000 Köln 1(DE)**

(54) **Katheterset für die Spinalanästhesie.**

(57) In eine Epiduralkanüle (10), die bis an die Dura vorgetrieben werden kann, wird eine Spinalkanüle (16) eingeschoben, deren Ende intrathekal placiert wird, wobei die Spinalkanüle (16) die Dura durchstößt. Dann wird ein Führungsdraht (21) durch die Spinalkanüle (16) hindurch nach intrathekal eingeschoben. Nach dem Entfernen der Spinalkanüle (16) wird über dem Führungsdraht (21) und innerhalb der Epiduralkanüle (10) ein Spinalkatheter (23) vorgeschoben. Die Epiduralkanüle (10) verleiht dem Katheter (23) beim Vorschieben die notwendige Richtungsstabilität und erleichtert das Durchdringen der bereits von der Spinalkanüle (16) perforierten Dura.

FIG. 1

## Katheterset für die Spinalanästhesie

Die Erfindung betrifft ein Katheterset für die Spinalanästhesie nach dem Oberbegriff des Anspruchs 1.

Spinalkatheter werden bisher durch eine Kanüle intrathekal placiert. Dabei erfolgt mit einer Spinal- oder Epiduralkanüle eine Punktion der Dura. Anschließend wird durch die Epiduralkanüle hindurch ein Spinalkatheter intrathekal eingeführt. Eine Schwierigkeit besteht darin, daß die Spinal- oder Epiduralkanüle einen kleinen Außendurchmesser haben muß, um die Größe der Duraverletzung und daraus entstehenden postspinalen Kopfschmerzen so gering wie möglich zu halten. Andererseits muß die Spinal- oder Epiduralkanüle einen so großen Durchmesser haben, daß der Spinalkatheter hindurchgeführt werden kann. Für sehr dünne Spinalkatheter gibt es jedoch keine Konnektoren, die eine dichte Katheter-Konnektorverbindung ermöglichen.

Aufgrund der genannten Schwierigkeiten werden derzeit Spinal- oder Epiduralkanülen benutzt, bei denen der Außendurchmesser größer ist als 0,65 mm (23 G).

Der Erfindung liegt die Aufgabe zugrunde, ein Katheterset der im Oberbegriff des Anspruchs 1 angegebenen Art zu schaffen, mit dem bei der Punktion der Dura eine möglichst kleine Duraöffnung erzeugt wird und bei dem dennoch ein verhältnismäßig dicker Spinalkatheter benutzbar ist.

Die Lösung dieser Aufgabe erfolgt erfindungsgemäß mit den im kennzeichnenden Teil des Anspruchs 1 angegebenen Merkmalen.

Mit dem erfindungsgemäßen Katheterset wird zunächst die Epiduralkanüle bis zur Außenwand der Dura vorgeschoben. Danach wird in die Epiduralkanüle die dünnere Spinalkanüle eingeführt, um mit dieser die Dura zu perforieren. Wenn die Spinalkanüle den Spinalraum erreicht hat, wird der Mandrin der Spinalkanüle herausgezogen, der Führungsdraht wird in die Spinalkanüle eingeschoben, die Spinalkanüle über den Führungsdraht herausgezogen und über den Führungsdraht wird der Spinalkatheter geschoben. Das Einführen des Katheters über den Führungsdraht erfolgt in ähnlicher Weise wie bei der bekannten Seldinger-Technik ein Katheter in ein Blutgefäß eingeführt wird. Nachdem die Katheterspitze den Spinalraum erreicht hat, werden Führungsdraht und Epiduralkanüle zurückgezogen. Die relativ dicke Epiduralkanüle diente dazu, den Perforationskanal durch das Muskelgewebe hindurch offenzuhalten und dem Katheter beim Vorschieben die notwendige Richtungsstabilität zu geben. Ferner umgibt die Epiduralkanüle die Spinalkanüle, während diese eingeführt wird. Das Vorschieben der Spinalkanüle wird daher nicht durch umgebendes Gewebe beeinträchtigt. Erst wenn die Spitze der Spinalkanüle gegen die Dura stößt, tritt ein deutlich spürbarer Widerstand auf. Die Perforation der Dura kann nachfolgend durch vorsichtiges Weiterschieben der Spinalkanüle behutsam vorgenommen werden.

Ein wesentlicher Vorteil der Erfindung besteht darin, daß die Perforationsstelle der Dura mit geringstmöglichem Durchmesser erzeugt wird, wobei der Katheter erst in die Perforationsstelle eingeschoben wird, nachdem die Spinalkanüle, mit der die Perforation durchgeführt wurde, zurückgezogen ist. Hierdurch ist es möglich, in die Perforationsöffnung einen Katheter einzuschieben, der diese Öffnung vollständig ausfüllt. Die Duraverletzung wird somit so gering wie möglich gehalten.

Vorzugsweise enthält die Spinalkanüle einen herausziehbaren Mandrin. Dieser Mandrin befindet sich bei der Perforation des Spinalraums in der Spinalkanüle. Nach erfolgter Perforation wird der Mandrin herausgezogen. Anhand des rückfließenden Liquor kann der Spinalraum identifiziert werden.

Der verwendete Führungsdraht ist sehr dünn (etwa 0,25 mm) und hat vorzugsweise eine flexible Spitze. Er kann auch mit Kunststoff überzogen sein. Der Führungsdraht wird durch die Spinalkanüle hindurchgeschoben und dient nach dem Herausziehen der Spinalkanüle als Führungselement für den durch die Epiduralkanüle hindurchgeschobenen Spinalkatheter.

Mit dem Merkmal des Anspruchs 3 wird das Einschieben des Spinalkatheters in das Kanülenrohr der Epiduralkanüle erleichtert. Obwohl das Kanülenrohr am rückwärtigen Ende leicht trichterförmig aufgebogen ist, paßt es in den Ansatz einer Spritze, die an die Epiduralkanüle angeschlossen werden kann.

Im folgenden wird unter Bezugnahme auf die Zeichnungen ein Ausführungsbeispiel der Erfindung näher erläutert.

Es zeigen:

Fig. 1 eine Darstellung der Komponenten des Kathetersets und

Fign. 2 bis 7 die verschiedenen Stadien der Benutzung des Katehtersets.

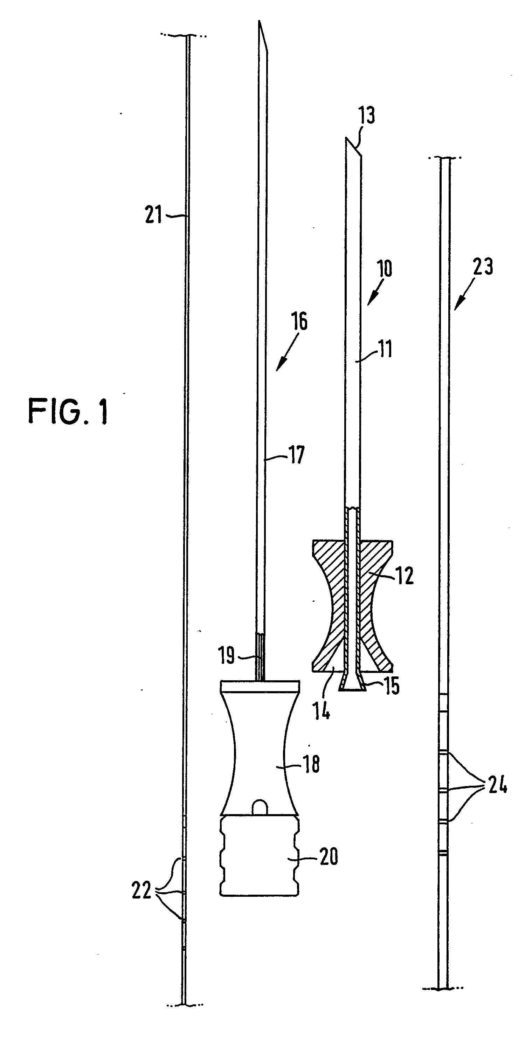

Gemäß Fig. 1 ist eine Epiduralkanüle 10 vorgesehen, die aus einem Kanülenrohr 11 und einem Griffstück 12 (oder Kanülenansatz) besteht. Diese Epiduralkanüle wird auch als Crawford-Kanüle bezeichnet. Das Kanülenrohr weist an seiner vorderen Spitze einen für die Gewebepunktion geeigneten Schliff 13 auf. Die Epiduralkanüle 10 kann z.B. Abmessungen von 0,7 x 0,9 x 80 mm haben, wobei die erste Zahl den Innendurchmesser, die zweite Zahl den Außendurchmesser und die dritte Zahl die freie Länge des Kanülenrohrs angibt.

Am rückwärtigen Ende des Griffstücks 12 befindet sich eine trichterförmige Mulde 14. Das rückwärtige Ende des Kanülenrohrs 11 ragt durch die Mulde 14 hindurch und erstreckt sich noch aus dieser heraus. Am rückwärtigen Ende des Kanülenrohrs 11 ist eine trichterförmige Erweiterung 15 vorgesehen, um das Einführen des Spinalkatheters zu erleichtern. Mit der Mulde 14 ist das Griffstück 12 als Anschlußelement zum Ansetzen einer (nicht dargestellten) Spritze ausgebildet.

Die Spinalkanüle 16 hat Abmessungen von z.B. 0,35 x 0,55 x 120 mm, wobei die verschiedenen Zahlen die gleichen Bedeutungen haben wie bei der zuvor erläuterten Epiduralkanüle. Am rückwärtigen Ende des Kanülenrohrs 17 befindet sich ein Griffstück 18. In der Spinalkanüle 16 sitzt ein Mandrin 19, der sich durch das Griffstück 18 hindurch erstreckt und am rückwärtigen Ende einen Handgriff 20 aufweist.

Zum Katheterset gehört ferner der Führungsdraht 21 mit flexibler Spitze. Dieser Führungsdraht hat Abmessungen z.B. von 0,25 x 900 mm, wobei die erste Zahl den Durchmesser und die zweite Zahl die Länge angibt. Am Führungsdraht 21 sind Markierungen 22 angebracht, um die Vorschublänge feststellen zu können.

Der Spinalkatheter 23 besteht aus einem langgestreckten flexiblen Katheterschlauch mit Abmessungen von 0,30 x 0,60 x 600 mm. Der Außendurchmesser des Spinalkatheters ist im wesentlichen gleich demjenigen der Spinalkanüle. In der Nähe des rückwärtigen Endes befinden sich Markierungen 24, um die Einschublänge kontrollieren zu können.

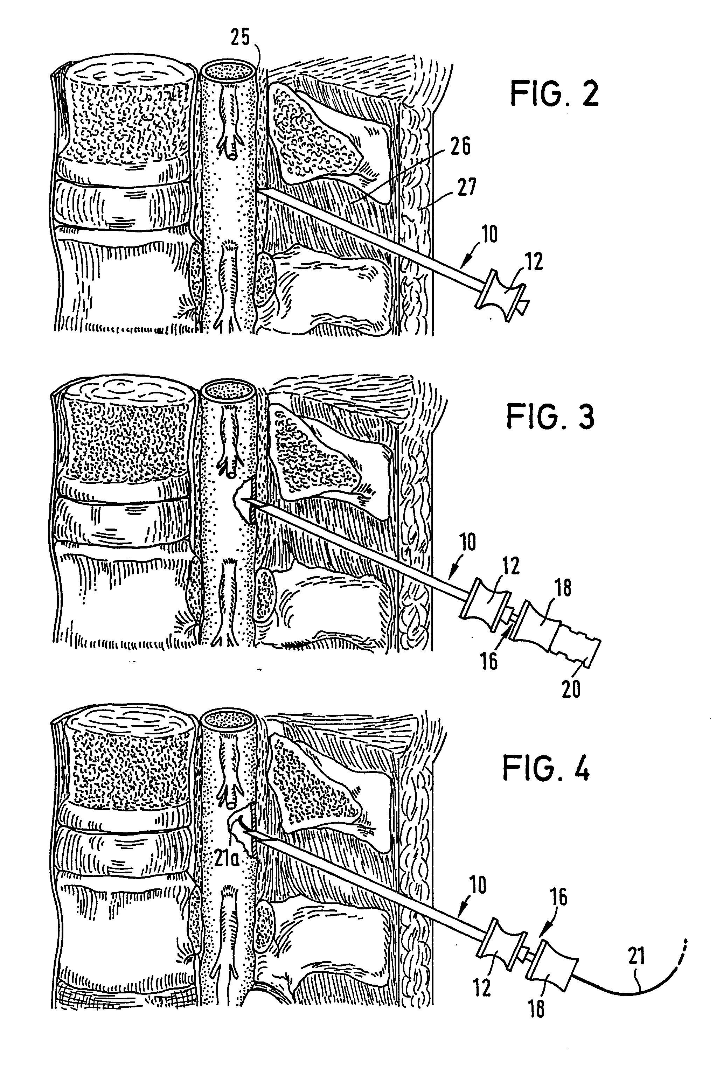

Die Anwendung des beschriebenen Kathetersets ist aus den Fign. 2 bis 7 erkennbar.

Zunächst werden mit der Epiduralkanüle 10 die Haut 27 und das Muskelgewebe 26 perforiert, bis der Schliff der Epiduralkanüle 10 an der Außenseite der Dura 25 anliegt.

Gemäß Fig. 3 wird die Spinalkanüle 16 mit dem in ihr befindlichen Mandrin 19 in die Epiduralkanüle bis nach intrathekal eingeschoben. Dabei durchdringt das vordere Ende der Spinalkanüle die Dura 25.

Fig. 4 zeigt, wie der Führungsdraht 21 durch die in der Epiduralkanüle liegende Spinalkanüle 16 hindurchgeschoben wird, nachdem zuvor der Mandrin 19 herausgezogen worden ist. Das weiche Ende 21a des Führungsdrahtes 21 tritt aus dem vorderen Ende der die Dura durchdringenden Spinalkanüle aus.

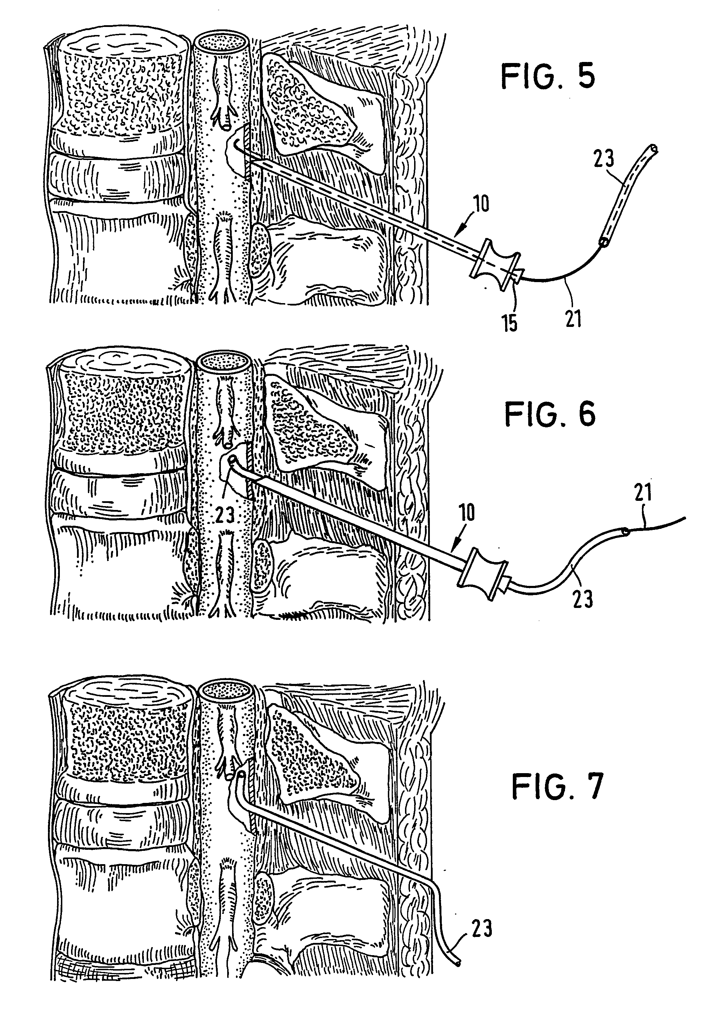

Nachdem die Spinalkanüle 16 über das rückwärtige Ende des Führungsdrahtes 21 hinweg zurückgezogen und aus der Epiduralkanüle 10 entfernt worden ist, wird gemäß Fig. 5 der Katheter 23 auf den Führungsdraht 21 aufgeschoben und über diesem in die Epiduralkanüle 10 eingeschoben. Hierbei erleichtert die trichterförmige Aufweitung 15 das Einführen des Katheters in das rückwärtige Ende des Kanülenrohrs 11.

Nachdem der Katheter intrathekal liegt, wird der Führungsdraht 21 gemäß Fig. 6 aus dem Katheter herausgezogen. Anschließend wird gemäß Fig. 7 über dem Spinalkatheter 23 die Epiduralkanüle 10 entfernt. Erforderlichenfalls kann der Katheter getunnelt werden. Schließlich wird an das rückwärtige Katheterende ein (nicht dargestellter) Konnektor angeschlossen, um eine intrathekale Injektion zu ermöglichen.

## Ansprüche

1. Katheterset für die Spinalanästhesie, mit einer Epiduralkanüle (10), deren Innendurchmesser kleiner als 1,0 mm ist, und einem durch die Epiduralkanüle (10) schiebbaren Spinalkatheter (23), **gekennzeichnet durch** eine in die Epiduralkanüle (10) einschiebbare Spinalkanüle (16), die im eingeschobenen Zustand mit ihrem vorderen Ende aus der Epiduralkanüle heraus vorsteht, und einen durch die Spinalkanüle (16) schiebbaren Führungsdraht (21), dessen Durchmesser kleiner ist als der Innendurchmesser des Spinalkatheters (23).

2. Katheterset nach Anspruch 1, dadurch gekennzeichnet. daß die Spinalkanüle (16) einen herausziehbaren Mandrin (19) enthält.

3. Katheterset nach Anspruch 1 oder 2, dadurch gekennzeichnet, daß die Epiduralkanüle (10) ein Griffstück (12) aufweist und daß das Kanülenrohr (11) am rückwärtigen Ende aus dem Griffstück (12) vorsteht und dort mit einer Aufweitung (15) versehen ist.

4. Katheterset nach Anspruch 3, dadurch gekennzeichnet, daß das Kanülenrohr (11) aus einer Mulde (14) am rückwärtigen Ende des Griffstückes (12) vorsteht.

5. Katheterset nach einem der Ansprüche 1 bis 4, dadurch gekennzeichnet, daß der Außendurchmesser der Spinalkanüle (16) kleiner ist als 0,6 mm.

6. Katheterset nach einem der Ansprüche 1 bis 5, dadurch gekennzeichnet, daß der Innendurchmesser der Epiduralkanüle etwa 0,7 mm beträgt.

7. Katheterset nach einem der Ansprüche 1 bis 6, dadurch gekennzeichnet, daß die Länge des Kanülenrohrs (11) der Epiduralkanüle (10) etwa 80 mm beträgt.

8. Katheterset nach einem der Ansprüche 1 bis 7, dadurch gekennzeichnet, daß die Spinalkanüle (16) im eingeschobenen Zustand etwa 10 bis 20 mm aus der Epiduralkanüle heraus vorsteht.

9. Katheterset nach einem der Ansprüche 1 bis 8, dadurch gekennzeichnet, daß das Griffstück (12) der Epiduralkanüle (10) als Anschlußelement zum Ansetzen einer Spritze ausgebildet ist.

FIG. 1

FIG. 2

FIG. 3

FIG. 4

FIG. 5

FIG. 6

FIG. 7

Europäisches
Patentamt

**EUROPÄISCHER RECHERCHENBERICHT**

Nummer der Anmeldung

EP 89112699.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | <u>DE - A1 - 3 327 585</u><br>(J.M.EVANS)<br>  * Fig. 1,2,5-11; Seite 7, Zeilen 1-7; Seite 8, 1. Absatz; Seite 9, 3. Absatz - Seite 11, 3. Absatz * | 1,2,7-9 | A 61 M 19/00<br>A 61 M 25/01<br>A 61 M 5/14 |
| A | -- | 3,5,6 | |
| Y | ACTA RADIOLOGICA, Band 39, Jänner-Juni 1953, Stockholm S.I.SELDINGER "Catheter replacement of the needle in percutaneous arteriography" Seiten 368-376<br>  * Seiten 370-371, 4. Absatz * | 1,2,7-9 | |
| | -- | | |
| A | <u>US - A - 2 922 420</u><br>(P.A.CHENG)<br>  * Fig. 1-4,7,8; Spalte 2, Zeile 66 - Spalte 3, Zeilen 4,20-23; Spalte 4, Zeilen 34-50; Spalte 4, Zeile 75 - Spalte 5, Zeile 3 * | 1-3,9 | RECHERCHIERTE SACHGEBIETE (Int. Cl.4)<br><br>A 61 B 17/00<br>A 61 M 5/00<br>A 61 M 25/00<br>A 61 M 19/00 |
| | ---- | | |

Der vorliegende Recherchenbericht wurde für alle Patentansprüche erstellt.

| Recherchenort | Abschlußdatum der Recherche | Prüfer |
|---|---|---|
| WIEN | 10-01-1990 | LUDWIG |